# EUROPEAN PATENT APPLICATION

(11) **EP 4 385 628 A1**
(43) Date of publication of application: **19.06.2024**
(21) Application number: 22855872.2
(22) Date of filing: 08.08.2022
(51) Int. Cl.: B05B 17/06, A01N 25/02, A01N 59/08, A61L 9/01, A61L 9/14

(54) **HYPOCHLOROUS ACID WATER SPRAY DEVICE**

(30) Priority: 11.08.2021 JP 2021131286
(71) Applicant: Nipro Corporation, Settsu-shi, Osaka 566-8510 (JP)
(72) Inventor: SANO, Yoshihiko, Settsu-shi, Osaka 566-8510 (JP); SUDO, Yoshinaga, Settsu-shi, Osaka 566-8510 (JP); MITSUHASHI, Makoto, Settsu-shi, Osaka 566-8510 (JP)
(74) Representative: Botti & Ferrari S.p.A.
(86) International application number: PCT/JP2022/030315
(87) International publication number: WO 2023/017810

(57) **Abstract**

Provided is a hypochlorous acid water spray device that utilizes ultrasonic spraying and inhibits decomposition of hypochlorous acid. The present invention relates to a hypochlorous acid water (7) spray device (100) including a storage unit (1) that stores hypochlorous acid water (7), an atomization mechanism (2) that atomizes the hypochlorous acid water (7) using ultrasonic vibration, and a guiding mechanism (3) that guides the hypochlorous acid water (7) to the atomization mechanism (2) by capillary action.

## Description

### TECHNICAL FIELD

The present invention relates to a spray device that utilizes ultrasonic vibration and is intended for atomizing and spraying hypochlorous acid water, and more particularly to the spray device that inhibits decomposition of hypochlorous acid.

### BACKGROUND ART

Conventionally, sodium hypochlorite as one of chlorine-based halogens exerts an immediate germ-killing potency at a very low concentration against bacteria, and is widely used in various fields including public health such as disinfection and sterilization in daily life, food, and medical care as the most reliable disinfectant in terms of the effect against viruses such as HIV and HBV. In particular, as the most familiar example, as for viruses having no envelope, such as viruses belonging to the family Caliciviridae including norovirus and SLV (sapovirus), and rotavirus, it is difficult to expect sterilization by alcohol disinfection, and chloric acid-based disinfection and sterilization are the first choice. Specifically, aqueous solutions containing sodium hypochlorite are used in the form of a spray or the like for disinfection and germ-killing/sterilization of various articles such as clothes, tableware, glass containers, and plastic containers, and environments, in homes and medical settings. In addition, spatial sterilization using hypochlorous acid water instead of a strongly alkaline aqueous sodium hypochlorite solution has also been performed.

Hypochlorous acid is regarded to have a more potent germ-killing and antiviral effect than that of sodium hypochlorite by a factor of 80 or more and have a wider antimicrobial spectrum covering non-enveloped viruses and fungi (Non-Patent Document 1). In addition, hypochlorous acid water is acidic to slightly acidic unlike the alkaline aqueous sodium hypochlorite solution. As a device for spraying hypochlorous acid water, for example, Patent Document 1 describes a spray device capable of drifting a dry mist in an indoor space for a long time without unnecessarily increasing the indoor humidity by utilizing ultrasonic vibration and spraying an aqueous hypochlorous acid solution as a mist having a particle diameter of about 0.5 µm in the indoor space. In addition, Patent Document 2 describes a hypochlorous acid water spray device that ultra-finely atomizes hypochlorous acid water in a water tank container storing the hypochlorous acid water by ultrasonic vibration and sprays the hypochlorous acid water from a nozzle. The hypochlorous acid water spray device includes a tray-shaped ultra-finely atomizing and generating device that has an ultrasonic vibrator disposed on a bottom surface thereof and that is immersed and floats in the hypochlorous acid water in the water tank container.

### PRIOR ART DOCUMENTS

### Patent Documents

Patent Document 1: JP 2019-154884 A
Patent Document 2: JP 2020-062289 A

### Non-Patent Document

Non-Patent Document 1: "<Table 2> Germ-killing activity of acidic electrolytic water (hypochlorous acid water)", Functional Water Foundation, 2015, [online], [searched on April 23, 2020], Internet <URL: http://www.fwf.or.jp/kinousui.html>

### SUMMARY OF THE INVENTION

### PROBLEM TO BE SOLVED BY THE INVENTION

Hypochlorous acid is easily decomposed by external force (energy). For this reason, spraying by heat or pressure greatly decomposes hypochlorous acid, and therefore, ultrasonic spraying utilizing an ultrasonic element is employed. Unfortunately, decomposition of the hypochlorous acid water on the ultrasonic element (vibrator) is promoted by energy of ultrasonic vibration even if the hypochlorous acid water is not vaporized. Therefore, the devices described in Patent Documents 1 and 2 have a problem that since the hypochlorous acid water stays on the ultrasonic element for a long time, hypochlorous acid is decomposed with time and the concentration thereof decreases.

Therefore, an object of the present invention is to provide a hypochlorous acid water spray device that utilizes ultrasonic vibration and is capable of inhibiting decomposition of hypochlorous acid.

### MEANS TO SOLVE THE PROBLEM

As a result of study of the above-mentioned problem, the present inventors have found that it is possible to provide a hypochlorous acid water spray device in which the direct influence of an ultrasonic vibrator on hypochlorous acid is reduced by utilization of capillary action so that decomposition of hypochlorous acid is inhibited, thereby completing the present invention.

That is, the present invention relates to:
[1] A hypochlorous acid water spray device including:
   a storage unit that stores hypochlorous acid water;
   an atomization mechanism that atomizes the hypochlorous acid water using ultrasonic vibration; and
   a guiding mechanism that guides the hypochlorous acid water to the atomization mechanism by capillary action;
[2] The hypochlorous acid water spray device according to [1], wherein liquid contact members, which include the storage unit and the guiding mechanism and come into contact with hypochlorous acid water, are free of any attached component that decomposes hypochlorous acid;
[3] The hypochlorous acid water spray device according to [1] or [2], further including an airflow generating mechanism that sprays atomized misty hypochlorous acid water upward by generating an upward airflow;
[4] The hypochlorous acid water spray device according to any one of [1] to [3], wherein misty hypochlorous acid water atomized in the atomization mechanism has an average particle diameter of 3 to 10 µm, preferably 3 to 7 µm, and more preferably 4 to 5 µm; and
[5] The hypochlorous acid water spray device according to any one of [1] to [4], wherein an ultrasonic wave used in the atomization mechanism has a frequency in a range of 1.0 to 3.0 MHz, preferably 1.0 to 2.4 MHz, and more preferably 1.2 to 1.6 MHz.

### EFFECTS OF THE INVENTION

According to the present invention, in a hypochlorous acid water spray device including an atomization mechanism that atomizes hypochlorous acid water using ultrasonic vibration, a guiding mechanism that utilizes capillary action for guiding the hypochlorous acid water from a storage unit to the atomization mechanism is provided, so that unnecessary decomposition of hypochlorous acid can be inhibited.

### BRIEF DESCRIPTION OF THE DRAWINGS

FIG. 1 is a schematic diagram illustrating a hypochlorous acid water spray device according to Embodiment 1.
FIG. 2 is a schematic diagram illustrating the hypochlorous acid water spray device according to Embodiment 2.

### EMBODIMENTS FOR CARRYING OUT THE INVENTION

Next, embodiments of the hypochlorous acid water spray device of the present invention are specifically described with reference to drawings, but the present invention is not intended to be limited to the embodiments. In addition, members, materials, and the like described below can be variously modified within the scope of the gist of the present invention. Furthermore, in the drawings, the same reference signs denote equivalent or same components, and sizes, positional relations, and the like of the structural elements are illustrated for convenience, and do not necessarily reflect actual conditions.

### (Embodiment 1)

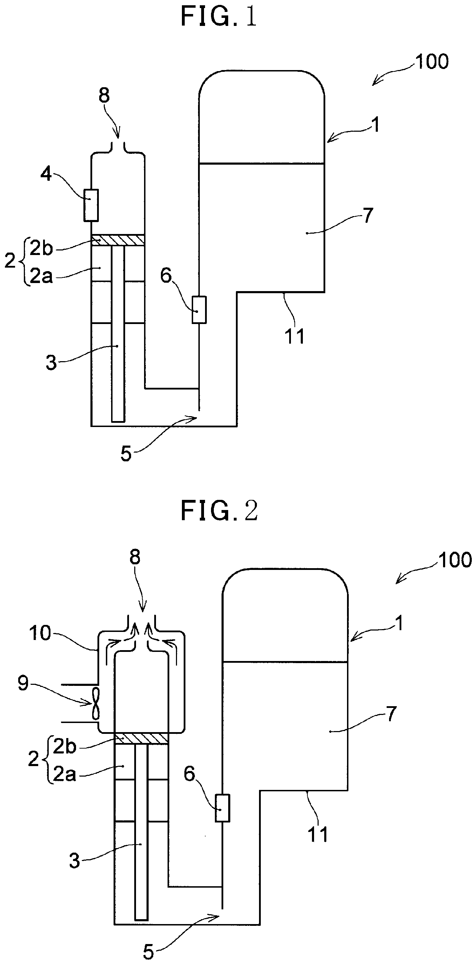

As shown in FIG. 1, a hypochlorous acid water 7 spray device 100 according to Embodiment 1 includes a storage unit 1 that stores hypochlorous acid water 7, an atomization mechanism 2 that atomizes the hypochlorous acid water 7 using ultrasonic vibration, and a guiding mechanism 3 that guides the hypochlorous acid water 7 from the storage unit 1 to the atomization mechanism 2 by capillary action. The hypochlorous acid water 7 spray device 100 may further include, as necessary, a spray mechanism 4 that sprays the atomized misty hypochlorous acid water 7. The hypochlorous acid water 7 is supplied from the storage unit 1 to a region where the guiding mechanism 3 is disposed through an outlet 5, and is guided to above the atomization mechanism 2, that is, above a vibration plate 2b by capillary action of the guiding mechanism 3. Accordingly, the hypochlorous acid water 7 spray device 100 has a structure that can reduce the amount of hypochlorous acid water staying on a vibrating element 2a and the vibration plate 2b to reduce the direct influence of the atomization mechanism 2 on hypochlorous acid.

The storage unit 1 is provided with the outlet 5 for the hypochlorous acid water 7 and an air inlet 6 for introducing air into the storage unit 1. The air inlet 6 is disposed above the outlet 5 with a height difference. This structure allows air to easily enter from the air inlet 6. The size of the outlet 5 is designed to be as small as possible, and the air inlet 6 is provided with a filter or the like so as to be also designed to have small openings. As a result, in this structure, small air bubbles enter the storage unit 1 from the air inlet 6, and the up-and-down width of the water surface in the storage unit 1 is made small to avoid generation of impact and kinetic energy, thereby inhibiting decomposition of hypochlorous acid. In consideration of such a mechanism, the air inlet 6 is preferably provided at a position lower than a bottom surface 11 of the storage unit 1 and higher than the outlet 5.

As the storage unit 1, a glass or resin tank or the like can be used. As for the member that constitutes the storage unit, a material such as glass, a PET resin, a PMMA resin, an ABS resin, an ASA resin, or a styrene-based resin (such as GPPS and HIPS) is used. Since there may be a possibility to promote decomposition of hypochlorous acid and decrease the hypochlorous acid concentration depending on material, a PET resin, a PMMA resin, an ABS resin, a styrene-based resin (such as GPPS and HIPS), or the like is preferably used.

The atomization mechanism 2 can include, for example, the ultrasonic element 2a including an ultrasonic vibrator and the vibration plate 2b. Since the vibration plate 2b is porous and has a lower surface in contact with the guiding mechanism 3 described later, hypochlorous acid water sucked up by capillary action of the guiding mechanism 3 reaches the vibration plate 2b.

As for the ultrasonic element 2a, when the ultrasonic wave used has a frequency in a low range, the vibrator has a large amplitude, and large energy is given to the hypochlorous acid water. Therefore, the frequency range used is preferably high. Meanwhile, when the ultrasonic wave used has a frequency in too high a range, the atomized misty hypochlorous acid water has a smaller average particle diameter, and hypochlorous acid tends to be easily decomposed. Therefore, the frequency range used can be appropriately selected in consideration of these tendencies. The frequency range used is not particularly limited, but is, for example, preferably 1.0 MHz or more, more preferably 1.2 MHz or more, and is preferably 3.0 MHz or less, more preferably 2.4 MHz or less, still more preferably 1.6 MHz or less.

In order to stabilize an atomized state of the misty hypochlorous acid water atomized in the atomization mechanism 2, the average particle diameter of the misty hypochlorous acid water is preferably small to some extent, and is preferably about 10 µm or less, more preferably 7 µm or less, and still more preferably 5 µm or less. The average particle diameter of the misty hypochlorous acid water atomized by the atomization mechanism 2 is preferably about 3 µm or more, and more preferably 4 µm or more from the viewpoint of inhibiting an increase in surface area to some extent and inhibiting decomposition of hypochlorous acid.

As described above, the guiding mechanism 3 guides the hypochlorous acid water 7 supplied from the outlet 5 of the storage unit 1 to the atomization mechanism 2 by capillary action so that the hypochlorous acid water 7 will not receive energy (acceleration) generated by ultrasonic vibration as much as possible. As for the guiding mechanism 3, for example, one that can replenish the hypochlorous acid water 7 supplied from the storage unit 1 to above the atomization mechanism 2 by capillary action, such as an elastic soft cloth or resin can be used. Specifically, materials such as a polyester-based resin, a PET resin, absorbent cotton, and a cellulose-based resin can be used, and a PET resin or the like is preferably used from the viewpoint of little influence on hypochlorous acid.

The spray mechanism 4 is not particularly limited as long as it can send misty particles of atomized hypochlorous acid water generated by the atomization mechanism 2 to the outside of the device. However, pressurization or a sending mechanism such as a fan can be used as the spray mechanism 4, and the spray mechanism 4 can be configured to spray the misty particles from a spout 8. Further, for example, in a case where the atomization mechanism 4 is provided in the spout 8, such as in a structure in which the atomization mechanism 4 and the spout 8 are connected together, it is also possible to spray and diffuse the misty particles simultaneously with vaporization (atomization) by the ultrasonic vibrator in the atomization mechanism 4.

In the spray device 100 described in Embodiment 1, as for liquid contact members with which the hypochlorous acid water 7 comes into direct contact, specifically, for example, the storage unit 1 and the guiding mechanism 3, it is preferable to use a member formed of a material having little influence on hypochlorous acid as described above. Furthermore, it is more preferable that the constituent members that constitute portions thereof are free of any attached component that decomposes hypochlorous acid. More specifically, it is particularly preferable that the portions in contact with hypochlorous acid water are free of any mold release agent for release from a mold used in manufacturing these structures (the storage unit 1 and the guiding mechanism 3), adhesive used in assembling the spray device 100, and the like attached thereto. Specifically, since it has been found by the present inventors that a siliconebased mold release agent reacts with hypochlorous acid water to deactivate the hypochlorous acid water, it is preferable to use these structures after sufficient washing when such a mold release agent remains. Typical examples of the component that decomposes hypochlorous acid include nitrogen contained in a polyamide-based resin.

In addition, the hypochlorous acid water spray device 100 according to Embodiment 1 may incorporate a balance (weight scale) for accurately controlling the spraying amount. For example, the weight of a storage tank or the entire device is measured by a weight sensor, and feedback control is performed or a decrease amount is displayed, whereby an accurate spraying amount can be recognized.

Moreover, the hypochlorous acid water spray device 100 according to Embodiment 1 may be provided with a thermometer, a CO₂ sensor, and the like. In order to prevent infection by coronavirus or influenza virus, closed, crowded, and close-contact settings should be avoided. As one method of controlling the timing for spraying the hypochlorous acid water, it is possible to detect a "closed, crowded, and close-contact" setting from a change in the temperature or CO₂ concentration, and spray the hypochlorous acid water. For this purpose, it is possible to provide a thermometer and a CO₂ sensor in the hypochlorous acid water spray device 100, and control the operation of the hypochlorous acid water spray device 100 in conjunction with the thermometer and the CO₂ sensor.

In the hypochlorous acid water spray device 100 according to Embodiment 1, the hypochlorous acid water 7 used preferably contains as few contaminant ions as possible other than hypochlorous acid molecules. Examples of the hypochlorous acid water containing as few contaminant ions as possible other than hypochlorous acid molecules include those in which 40 mol% or more of the total amount of chlorine in the liquid is chlorine of hypochlorous acid, that is, chlorine derived from hypochlorous acid. This is because if the hypochlorous acid water 7 contains a large amount of contaminant ions, hypochlorous acid and contaminant ions may cause a chemical reaction to promote decomposition in the process of evaporation and aggregation of moisture in the mist. For this reason, as for water used for preparing hypochlorous acid water, pure water, purified water, ion-exchanged water, or the like is preferably used in order to reduce contamination with unintended components, for example, metal ions and organic substances contained in tap water.

The hypochlorous acid water used is preferably electrolytic water. More specifically, the hypochlorous acid water is preferably electrolytic water containing, as a main component, hypochlorous acid and obtained by electrolysis using hydrochloric acid or sodium chloride as a raw material. As such hypochlorous acid water, a commercially available product can also be used.

The hypochlorous acid water used preferably has a pH of 3.2 or more, more preferably 3.5 or more, and particularly preferably 5.8 or more. Setting the pH of the hypochlorous acid water used to 3.2 or more can reduce corrosion and deterioration of parts used in the spray device, and further reduce generation of chlorine gas (hydrogen chloride gas).

Owing to properties such as not too low a pH and a strong germ-killing and antiviral potency, the hypochlorous acid water is suitably used for washing and sterilizing the skin as in hand/finger washing (hand washing sterilization), sterilizing indoor spaces, and the like in various environments, for example, hospitals, welfare facilities, hospitals, nursery schools, kindergarten, schools, and cram schools. In particular, in an aspect in which the hypochlorous acid water does not contain a salt and has a pH that is not too low, there is little concern about corrosion, and thus the hypochlorous acid water can be used in combination with a humidification function or an air cleaning function.

### (Embodiment 2)

The hypochlorous acid water 7 spray device 100 according to Embodiment 2 further includes an airflow generating mechanism that sprays atomized misty hypochlorous acid water upward by generating an upward airflow. As illustrated in FIG. 2, the spout 8 is covered with an outer peripheral portion 10 provided with a fan 9 for generating an upward airflow (arrows in the drawing). More specifically, in FIG. 2, the fan 9 and the outer peripheral portion 10 constitute an airflow generating mechanism that sprays atomized misty hypochlorous acid water upward. Here, the means for generating the airflow is not limited to the fan, but is intended to have a configuration capable of indirectly spraying atomized misty hypochlorous acid water upward by the generated upward airflow in conjunction with the outer peripheral portion 10 serving as a guide. The misty hypochlorous acid water 7 atomized by the atomization mechanism 2 utilizing an ultrasonic wave is usually ejected from the spout 8 to the outside by using the fan 9. The wind from the fan 9 that directly hits the misty hypochlorous acid water deactivates hypochlorous acid in the misty hypochlorous acid water, which may reduce the effective chlorine concentration in the ejected mist. As a countermeasure, as shown in FIG. 2, by generating an upward airflow outside to promote ejection of atomized misty hypochlorous acid water, the misty hypochlorous acid water is efficiently ejected upward, and deactivation of hypochlorous acid is satisfactorily remedied as compared with a case where the wind from the fan directly hits the hypochlorous acid water. The portions in FIG. 2 same as those in FIG. 1 are denoted by the same reference signs as those in FIG. 1, and the description thereof is omitted.

In the present description, the matters described in each of Embodiments 1 and 2 are also applied to the other embodiment as long as the object of the invention can be achieved and there is no contradiction. In addition, the spray device of the present invention can be used to sterilize various indoor spaces such as clinics, hospitals, welfare facilities, nursery schools, kindergartens, schools, cram schools, elderly housings, and standard homes.

### (Conclusion)

(1) A hypochlorous acid water spray device of the present invention includes a storage unit that stores hypochlorous acid water, an atomization mechanism that atomizes the hypochlorous acid water using ultrasonic vibration, and a guiding mechanism that guides the hypochlorous acid water to the atomization mechanism by capillary action. With this configuration, the amount of hypochlorous acid water staying on the atomization mechanism that atomizes the hypochlorous acid water using ultrasonic vibration from an ultrasonic vibrator, a vibration plate, or the like can be reduced as much as possible, the influence of the atomization mechanism on hypochlorous acid can be reduced, and unnecessary decomposition of hypochlorous acid can be inhibited.
(2) Liquid contact members, which include the storage unit and the guiding mechanism and come into contact with hypochlorous acid water, are preferably free of any attached component that decomposes hypochlorous acid. With this configuration, unnecessary decomposition (deactivation) of hypochlorous acid is inhibited.
(3) The hypochlorous acid water spray device of the present invention preferably further includes an airflow generating mechanism that sprays atomized misty hypochlorous acid water upward by generating an upward airflow. The airflow generating mechanism can more efficiently guide the atomized misty hypochlorous acid water to the outside while reducing direct impact on the atomized misty hypochlorous acid water and reducing the risk that the effective chlorine concentration in the ejected mist is reduced.
(4) The atomized misty hypochlorous acid water preferably has an average particle diameter of 3 to 10 µm, more preferably 3 to 7 µm, and still more preferably 4 to 5 µm. Setting the average particle diameter of the atomized misty hypochlorous acid water within such a range can stabilize the atomized state and inhibit decomposition of the hypochlorous acid water.
(5) An ultrasonic wave used in the atomization mechanism preferably has a frequency in a range of 1.0 to 3.0 MHz, more preferably 1.0 to 2.4 MHz, and still more preferably 1.2 to 1.6 MHz. Setting the frequency range used within such a range can provide, under moderate energy application, atomized misty hypochlorous acid water having an appropriate particle diameter with which decomposition is not excessively promoted.

### DESCRIPTION OF REFERENCE NUMERALS

- 100: SPRAY DEVICE
- 1: STORAGE UNIT
- 2: ATOMIZATION MECHANISM
- 2a: ULTRASONIC ELEMENT
- 2b: VIBRATION PLATE
- 3: GUIDING MECHANISM
- 31: NARROW TUBE
- 4: SPRAY MECHANISM
- 5: OUTLET
- 6: AIR INLET
- 7: HYPOCHLOROUS ACID WATER
- 8: SPOUT
- 9: FAN
- 10: OUTER PERIPHERAL PORTION

## Claims

1. A hypochlorous acid water spray device comprising:
a storage unit that stores hypochlorous acid water;
an atomization mechanism that atomizes the hypochlorous acid water using ultrasonic vibration; and
a guiding mechanism that guides the hypochlorous acid water to the atomization mechanism by capillary action.

2. The hypochlorous acid water spray device according to claim 1, wherein liquid contact members, which include the storage unit and the guiding mechanism and come into contact with hypochlorous acid water, are free of any attached component that decomposes hypochlorous acid.

3. The hypochlorous acid water spray device according to claim 1 or 2, further comprising an airflow generating mechanism that sprays atomized misty hypochlorous acid water upward by generating an upward airflow.

4. The hypochlorous acid water spray device according to claim 1 or 2, wherein misty hypochlorous acid water atomized in the atomization mechanism has an average particle diameter of 3 to 10 µm.

5. The hypochlorous acid water spray device according to claim 1 or 2, wherein an ultrasonic wave used in the atomization mechanism has a frequency in a range of 1.0 to 3.0 MHz.
